# EUROPEAN PATENT APPLICATION

(11) **EP 2 149 849 A1**
(43) Date of publication of application: **03.02.2010**
(21) Application number: 09005621.9
(22) Date of filing: 22.04.2009
(51) Int. Cl.: G06F 19/00

(54) **Method and apparatus for providing customized interface in ultrasound system**

(30) Priority: 25.07.2008 KR 20080072978
(71) Applicant: MEDISON CO., LTD., Gangwon-do 250-875 (KR)
(72) Inventor: Shin, Soo-Hwan, Seoul 137-061 (KR); Park, Sung-In, Gyeonggi-do 465-705 (KR); Lee, Su-Myeong, Gyeonggi-do 431-060 (KR); Kim, Yung-Gil, Seoul 137-932 (KR); Kim, Jae-Gyoung, Seoul 143-203 (KR)
(74) Representative: Schmid, Wolfgang

(57) **Abstract**

A method and apparatus for providing a customized interface in an ultrasound system is provided. The customized interface providing method includes: receiving a request for use of the ultrasound system from a user; displaying, in response to the request, a selection screen associated with selecting any one of software modes provided from the ultrasound system; receiving selection information from the user according to displaying of the selection screen; and executing a software mode corresponding to the selection information.

## Description

### BACKGROUND

### Field of the Invention

Embodiments of the present invention relate to a method and apparatus for providing a customized interface, and more particularly, to a method and apparatus for providing a customized interface that may change a software mode for each user in an ultrasound system.

### Description of the Related Art

An ultrasound system denotes a system that may emit ultrasound signals from the body surface of a subject to a select interior portion of the body and provide images associated with blood flow or a section of soft tissue using information associated with reflected ultrasound signals. The ultrasound system is generally small and inexpensive, and also provides a display in real time. In addition, the ultrasound system has no absorbed dose such as with X rays and the like, and thus is highly stable. The ultrasound system is being widely used together with other image diagnostic apparatuses such as an X-ray diagnostic apparatus, a computerized tomography (CT) scanner, a magnetic resonance image (MRI) apparatus, a nuclear medicine diagnostic apparatus, and the like.

As the ultrasound system continues to be developed, many functions are currently provided for users. Also, a software configuration of the ultrasound system becomes more complex.

Some users may skillfully use the complex ultrasound system, whereas many users may still not use all the functions that are provided from the complex ultrasound system. Although users may use all the functions, they may need to go through a complex process to control a desired function. Specifically, the users may need to go through many operation depths.

Also, since users generally use only limited functions of the ultrasound system, displaying all other functions besides the users' most used functions on a screen may decrease work efficiency. Accordingly, there is a need for a method and apparatus for providing a customized interface that may control functions.

### SUMMARY

According to an aspect of the present invention, there is provided a method of providing a customized interface in an ultrasound system, the method including: receiving a request for use of the ultrasound system from a user; displaying, in response to the request, a selection screen associated with selecting any one of software modes provided from the ultrasound system; receiving selection information from the user according to displaying of the selection screen; and executing a software mode corresponding to the selection information.

In an aspect of the present invention, the method may further include executing a previously executed software mode when the selection information is not received from the user.

Also, the executing of the software mode may include: receiving user environment information from the user; and reflecting the user environment information in the software mode corresponding to the selection information to thereby execute the software mode.

Also, the executing of the software mode may further include reflecting previously reflected user environment information in the software mode to thereby execute the software mode when the user environment information is not received from the user.

Also, the software mode may be classified according to any one of a proficiency level associated with a control of the ultrasound system and a function level of the ultrasound system.

Also, the software mode may be any one of an easy mode and an expert mode.

Also, the method may further include displaying a task pane window region associated with a control of the ultrasound system for the user via the executed software mode. The task pane window region may include a plurality of different information that is switched with each other according to a user selection, and the plurality of information may be associated with a particular function of the ultrasound information.

According to another aspect of the present invention, there is provided an apparatus for providing a customized interface in an ultrasound system, the apparatus including: a display unit to display a selection screen associated with selecting any one of software modes provided from the ultrasound system, in response to a request for use of the ultrasound system that is received from a user; an input unit to receive selection information from the user according to displaying of the selection screen; and an execution unit to execute a software mode corresponding to the selection information.

According to embodiments of the present invention, there may be provided a method and apparatus for providing a customized interface that may receive selection information associated with a software mode from a user and thereby more effectively provide a customized software mode.

Also, according to embodiments of the present invention, there may be provided a method and apparatus for providing a customized interface that may execute a software mode corresponding to selection information input from a user and thereby significantly reduce an operation depth.

Also, according to embodiments of the present invention, there may be provided a method and apparatus for providing a customized interface that may provide a task pane window region containing different pieces of information that may be switched with each other according to a user selection and thereby improve a user convenience and work efficiency.

Also, according to embodiments of the present invention, there may be provided a method and apparatus for providing a customized interface that enables a user to access and correct information using a task pane window region and thereby may reduce an operation depth and time.

### BRIEF DESCRIPTION OF THE DRAWINGS

These and/or other aspects, features, and advantages of the invention will become apparent and more readily appreciated from the following description of exemplary embodiments, taken in conjunction with the accompanying drawings of which:
- FIG 1: is a block diagram illustrating an apparatus for providing a customized interface according to an embodiment of the present invention;
- FIG 2: is a flowchart illustrating a method of providing a customized interface according to an embodiment of the present invention;
- FIG 3: is a flowchart illustrating a method of providing a customized interface according to another embodiment of the present invention;
- FIG 4: is an example of a screen being displayed by execution of a software mode according to an embodiment of the present invention; and
- FIG 5: is an example of a task pane window region according to an embodiment of the present invention.

### DETAILED DESCRIPTION

Reference will now be made in detail to exemplary embodiments of the present invention, examples of which are illustrated in the accompanying drawings, wherein like reference numerals refer to the like elements throughout. Exemplary embodiments are described below to explain the present invention by referring to the figures.

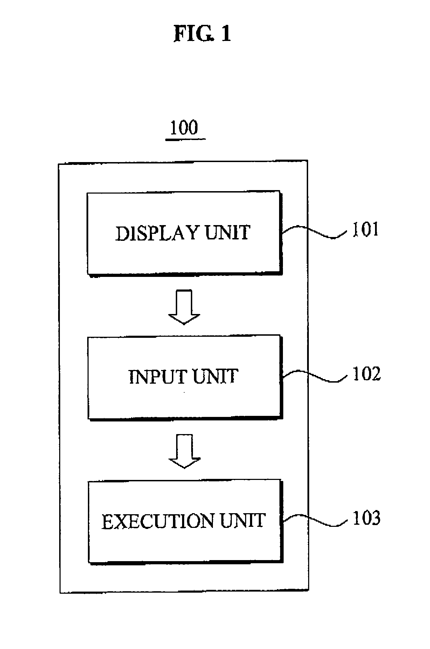

FIG 1 is a block diagram illustrating an apparatus 100 for providing a customized interface according to an embodiment of the present invention.

The customized interface providing apparatus 100 is included in an ultrasound system. The customized interface providing apparatus 100 may receive a request for use of the ultrasound system from a user that desires to use the ultrasound system. In response to the request, the customized interface providing apparatus 100 may display a selection screen that enables the user to select any one of software modes provided from the ultrasound system. The selection screen may be displayed via a predetermined display device installed in the ultrasound system. The user may select the user's desired software mode on the selection screen of the ultrasound system. The customized interface providing apparatus 100 may execute the selected software mode.

The customized interface providing apparatus 100 may provide the ultrasound system that may execute a customized software mode for each user and thereby improve a user's work efficiency and also reduce an operation depth. For example, the customized interface providing apparatus 100 may provide an expert mode for a user that is capable of using all the diversified functions of the ultrasound system and may also provide an easy mode for a user that is capable of using only limited functions.

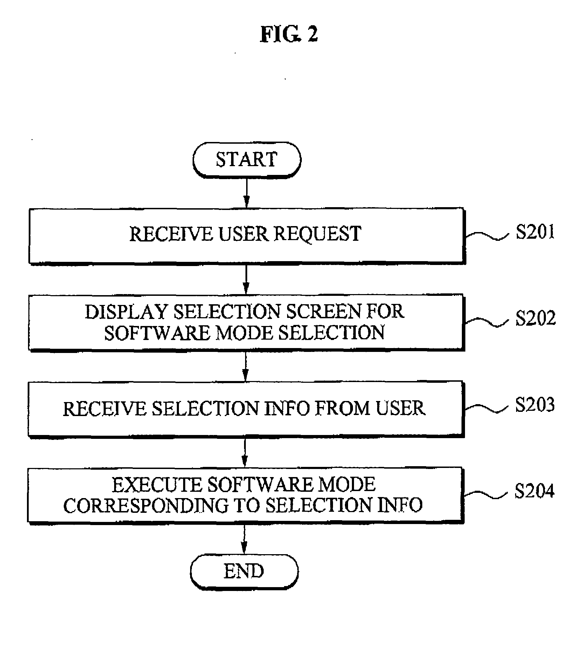

As shown in FIG. 1, the customized interface providing apparatus 100 includes a display unit 101, an input unit 102, and an execution unit 103. Hereinafter, an operating method of the customized interface providing apparatus 100 will be described in detail with reference to FIGS. 2 through 5.

FIG 2 is a flowchart illustrating a method of providing a customized interface according to an embodiment of the present invention.

As shown in FIG 2, the method may be performed via operations S201 through S204. In this instance, operations S201 and S202 may be performed by the display unit 101. Operation S203 may be performed by the input unit 102 and operation S204 may be performed by the execution unit 103.

In operation S201, the display unit 101 receives a request for use of an ultrasound system from a user. The request may be input via a predetermined input device connected to the ultrasound system. The input device may be, for example, a keyboard, a mouse, or a touch screen. The request may be performed by powering up the ultrasound system.

In operation S202, in response to the request, the display unit 101 displays a selection screen associated with selecting any one of various software modes that are provided from the ultrasound system. The selection screen may include a selection icon capable of indicating the software modes.

The software modes may be classified according to any one of a proficiency level associated with a control of the ultrasound system and a function level of the ultrasound system. The proficiency level may indicate an ability to control of the ultrasound system and be classified into an advance grade, an intermediate grade, and a beginner grade. The function level may denote a number of functions that the user may control in the user's selected software mode among all the functions provided from the ultrasound system.

According to an embodiment of the present invention, the software mode may be any one of an easy mode and an expert mode. The easy mode may be a software mode that consists of functions that are determined according to a number of usage frequencies of the user among all the functions of the ultrasound system. The expert mode may be a software mode that provides all the functions of the ultrasound system using an upper menu, and a lower menu provided according to a selection of the upper menu.

In operation S203, the input unit 102 receives selection information from the user according to displaying of the selection screen. The selection information may be generated when the user selects any one of the software modes, provided from the ultrasound system, using a predetermined input device. The input device may be, for example, a keyboard, a mouse, and a touch screen.

In operation S204, the execution unit 103 executes a software mode corresponding to the selection information. Specifically, the execution unit 103 may identify the selected software mode using the selected selection information and execute the identified software mode.

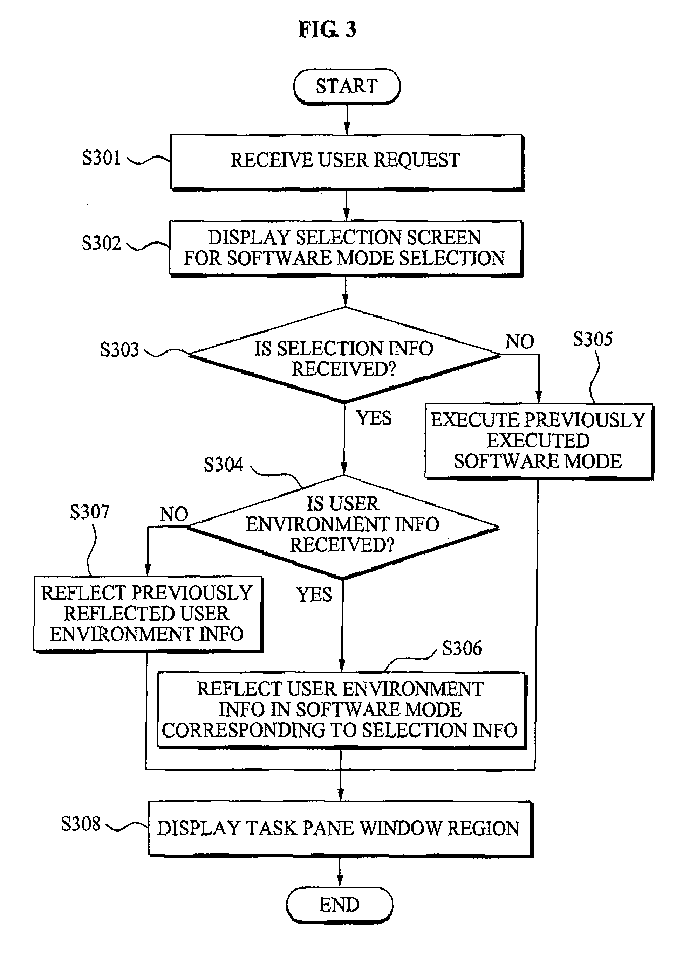

Hereinafter, operations S201 through S204 will be further described in detail with reference to FIGS. 3 through 5.

FIG 3 is a flowchart illustrating a method of providing a customized interface according to another embodiment of the present invention.

As shown in FIGS. 3, the method may be performed via operations S301 through S308. In this instance, operations S301, S302, and S308 may be performed by the display unit 101. Operation S303 may be performed by the input unit 102. Operations S304 through S307 may be performed by the execution unit 103.

In operation S301, the display unit 101 receives a request for use of an ultrasound system from a user. Operation S301 will be the same as or similar to the aforementioned operation S201 of FIG. 2 and thus further detailed descriptions related thereto will be omitted here.

In operation S302, in response to the request, the display unit 101 displays a selection screen associated with selecting any one of software modes that are provided from the ultrasound system. The selection screen may include a selection icon capable of identifying the software modes.

The software modes may be classified according to any one of a proficiency level associated with a control of the ultrasound system and a function level of the ultrasound system. The proficiency level may indicate an ability to control the ultrasound system and be classified into an advance grade, an intermediate grade, and a beginner grade. The function level may denote a number of functions that the user may control in the user's selected software mode among all the functions provided from the ultrasound system.

According to an embodiment of the present invention, the software mode may be any one of an easy mode and an expert mode.

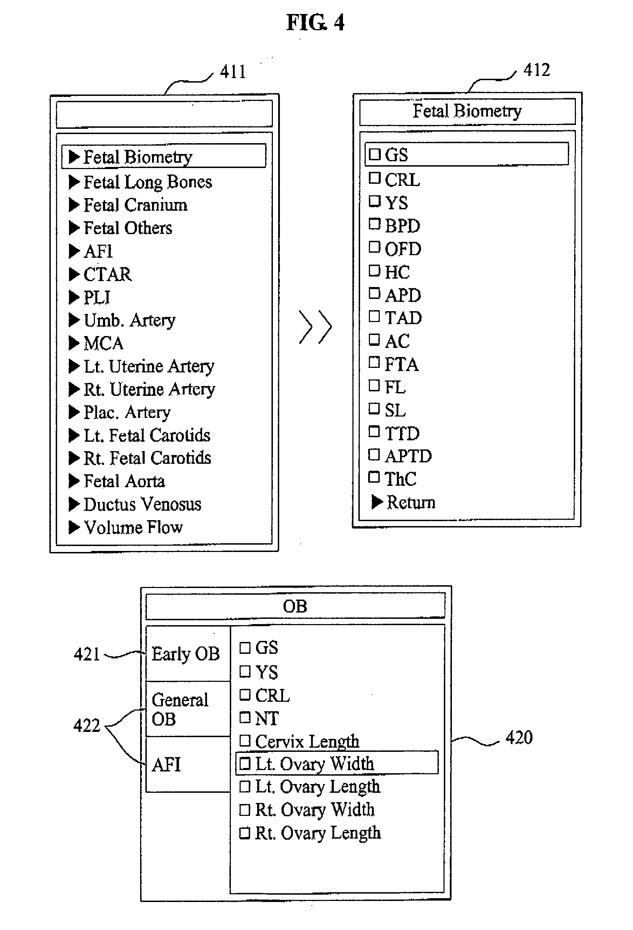

FIG 4 is an example of a screen being displayed by execution of a software mode according to an embodiment of the present invention. In particular, screens 411 and 412 may be provided when executing an expert made. A screen 420 may be provided when executing an easy mode.

In this instance, the expert mode may be a software mode that provides all the functions of the ultrasound system using an upper menu, and a lower menu provided according to a selection of the upper menu. Also, in the expert mode, the ultrasound system may initially display higher functions among all the functions that may be provided from the ultrasound system. When the user selects any one of the higher functions, the ultrasound system may display lower functions included in the selected higher function. For example, as shown in FIG. 4, when the user selects a higher function "fetal biometry" on the screen 411 where the higher functions are displayed, lower functions belonging to the selected higher function "fetal biometry" may be displayed on the screen 412.

Also, the easy mode may be a software mode that consists of functions that are determined according to a number of usage frequencies of the user among all the functions of the ultrasound system. In the easy mode, only functions generally used by users may be displayed, which is different from the expert mode where all the functions in relation to an application unit are displayed. Accordingly, unlike a two-depth configuration in the expert mode, functions may be constructed in the easy mode regardless of application. When the functions provided in the easy mode are insufficient, or when an unnecessary function is provided, the user may reconstruct the functions using a setting window. Also, in the easy mode, there is no need to exit and return to the higher functions and the lower functions as in the expert mode. Accordingly, the operation depth may be reduced. Also, the user may add, delete, or reconstruct a plurality of user pages.

Specifically, as shown in FIG. 4, the user may view the screen 420 consisting of only a portion of functions that are determined according to the number of usage frequencies. As necessary, the determined functions may be classified into an activated page 421 and an inactivated page 422 according to a user selection.

In operation S303, the input unit 102 receives selection information from the user according to displaying of the selection screen. The selection information may be generated when the user selects any one of the software modes, provided from the ultrasound system, using a predetermined input device. The input device may be, for example, a keyboard, a mouse, and a touch screen. As shown in FIG. 3, when the input unit 102 receives the selection information from the user, operation S304 may be performed. Conversely, when the input unit 102 does not receive the selection information or when the user selects to reject input of the selection information, operation S305 may be performed- For example, the rejecting operation may be performed by the user's selecting a "selection information input rejecting icon" included in the selection screen.

In operation S304, the execution unit 103 receives user environment information from the user. The user environment information may be unique setting information of the user that is applicable in the software mode selected by the user.

As shown in FIG. 3, when the user environment information is received from the user, operation S306 may be performed. Conversely, when the user environment information is not received, or when the user selects to reject input of the user environment information, operation S307 may be performed.

In operation S305, when the user does not input the selection information, the execution unit 103 executes a previously executed software mode. For example, when the user does not select a software mode or selects to reject input of selection information due to the continuous usage by the same user, the execution unit 103 may execute the user's last used software mode and thereby reduce an unnecessary procedure. According to an embodiment of the present invention, when executing the previously executed software mode, the execution unit 103 may reflect user environment information that was reflected during execution of the previous software mode.

In operation S306, the execution unit 103 reflects the user environment information in the software mode corresponding to the selection information to thereby execute the software mode. The user environment information may be unique setting information of the user that is applicable to the software mode selected by the user. Accordingly, the execution unit 103 may provide the user with a customized interface when the user's unique setting information is reflected in the selected software mode.

In operation 5307, when the user environment information is not received from the user, the execution unit 103 reflects the previously reflected user environment information in the software mode to thereby execute the software mode. For example, when the user does not select the user environment information or selects to reject input of the user environment information due to the continuous usage by the user, the execution unit 103 may reflect the user environment information last used by the user in the software mode and thereby reduce an unnecessary procedure. For example, when the user selects an easy mode among the software modes, the execution unit 103 may reflect user environment information reflected during execution of the easy mode to thereby execute the selected easy mode.

Here, although it is described that the user environment information is subject to each of the software modes in operations S304 through S307, the user environment information may be unique setting information that may be commonly applied regardless of types of the software modes. For examples, when a key setting of the input device is different for each user, the execution unit 103 may reflect, in the selected software mode, the user environment information associated with a key configuration of the input device, regardless of a type of the software mode.

In operation S308, the display unit 101 displays a task pane window region associated with a control of the ultrasound system via the executed software mode. The task pane window region may include a plurality of different information that is switched with each other according to a user selection. The plurality of information may be associated with a particular function of the ultrasound information.

The task pane window region may represent effective information for the users. Also, the users may perform an operation via the task pane window region without a need to popup a task pane window for each piece of information and to check and correct them. Accordingly, the user may control the ultrasound system with reduced operation depths.

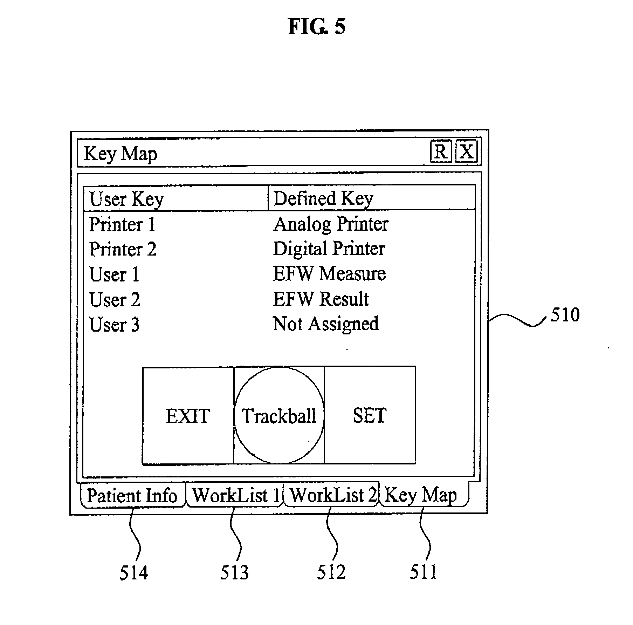

FIG 5 is an example of a task pane window region 510 according to an embodiment of the present invention. As shown in FIG 5, the display unit 101 may classify, into four contents, information associated with a particular function of an ultrasound system and display the classified information for a user via the task pane window region 510. The four contents may be patient information content 514, first work list content 513, second work list content 512, and key map content 511. In particular, the task pane window region 510 displays information according to the key map content 511 tap-selectcd by the user, that is, displays information that is classified according to the key map content 511.

The patient information content 514 includes specific information associated with a particular patient. Accordingly, the user may immediately correct information in the corresponding task pane window region without a need to popup an existing patient widow and thus may control the ultrasound system with reduced operation depths.

The first work list content 513 includes a previous clinic record of the particular patient. Accordingly, the user may immediately select and verify the previous clinic record in the task pane window region without a need to popup the patient window and thereby may control the ultrasound system with reduced operation depths.

The second work list content 512 includes a list of subsequent patients followed by a current patient. Accordingly, every time the patient changes, the user may not be required to input patient information and change a predetermined setting. Specifically, the user may immediately select a subsequent patient in the task pane window region and set a scan to be appropriate for the subsequent patient and thereby may control the ultrasound system with reduced operation depths.

The key map content 511 includes setting information associated with a user designated key, a print key, a set key, an exist key, and the like. Accordingly, the user may simply change a setting in the task pane window region without a need to popup a separate utility window and change the setting, and thereby may control the ultrasound system with reduced operation depths.

As described above, since users may perform operations for a plurality of contents and information included in each of the contents via a task pane window region without a need to popup a utility window corresponding to each piece of information and then to verify or correct the information, it is possible to control the ultrasound system with reduced operation depths.

The customized interface providing method according to the above-described exemplary embodiments of the present invention may be recorded in computer-readable media including program instructions to implement various operations embodied by a computer. The media may also include, alone or in combination with the program instructions, data files, data structures, and the like. Examples of computer-readable media include magnetic media such as hard disks, floppy disks, and magnetic tape; optical media such as CD ROM disks and DVDs; magneto-optical media such as optical disks; and hardware devices that are specially configured to store and perform program instructions, such as read-only memory (ROM), random access memory (RAM), flash memory, and the like. Examples of program instructions include both machine code, such as produced by a compiler, and files containing higher level code that may be executed by the computer using an interpreter. The described hardware devices may be configured to act as one or more software modules in order to perform the operations of the above-described exemplary embodiments of the present invention, or vice versa.

Although a few exemplary embodiments of the present invention have been shown and described, the present invention is not limited to the described exemplary embodiments. Instead, it would be appreciated by those skilled in the art that changes may be made to these exemplary embodiments without departing from the principles and spirit of the invention, the scope of which is defined by the claims and their equivalents.

## Claims

1. A method of providing a customized interface in an ultrasound system, the method comprising:
receiving a request for use of the ultrasound system from a user;
displaying, in response to the request, a selection screen associated with selecting any one of software modes provided from the ultrasound system;
receiving selection information from the user according to displaying of the selection screen; and
executing a software mode corresponding to the selection information.

2. The method of claim 1, further comprising:
executing a previously executed software mode when the selection information is not received from the user.

3. The method of claim 1, wherein the executing of the software mode comprises:
receiving user environment information from the user; and
reflecting the user environment information in the software mode corresponding to the selection information to thereby execute the software mode.

4. The method of claim 3, wherein the executing of the software mode further comprises reflecting previously reflected user environment information in the software mode to thereby execute the software mode when the user. environment information is not received from the user.

5. The method of claim 1, wherein the software mode is classified according to any one of a proficiency level associated with a control of the ultrasound system and a function level of the ultrasound system.

6. The method of claim 1, wherein the software mode is any one of an easy mode and an expert mode.

7. The method of claim 6, wherein the easy mode is a software mode that includes a portion of functions that are determined according to a number of usage frequencies of the user among all the functions of the ultrasound system.

8. The method of claim 6, wherein the expert mode is a software mode that constructs all the functions of the ultrasound system using an upper menu and a lower menu according to a selection of the upper menu.

9. The method of claim 1, further comprising:
displaying a task pane window region associated with a control of the ultrasound system for the user via the executed software mode.

10. The method of claim 9, wherein:
the task pane window region includes a plurality of different information that is switched with each other according to a user selection, and
the plurality of information is associated with a particular function of the ultrasound information.

11. A computer-readable recording medium storing a program for implementing the method of claim 1.

12. An apparatus for providing a customized interface in an ultrasound system, the apparatus comprising:
a display unit to display a selection screen associated with selecting any one of software modes provided from the ultrasound system, in response to a request for use of the ultrasound system that is received from a user;
an input unit to receive selection information from the user according to displaying of the selection screen; and
an execution unit to execute a software mode corresponding to the selection information.

13. The apparatus of claim 12, wherein, when the selection information is not received from the user, the execution unit executes a previously executed software mode.

14. The apparatus of claim 12, wherein the execution unit receives user environment information from the user, and reflects the user environment information in the software mode corresponding to the selection information to thereby execute the software mode.

15. The apparatus of claim 12, wherein the display unit displays the selection screen associated with selecting any one of the software modes provided from the ultrasound system, in response to the request for use of the ultrasound system that is received from the user and displays a task pane window region associated with a control of the ultrasound system for the user via the executed software mode.
